# EUROPEAN PATENT APPLICATION

(11) **EP 3 593 733 A1**
(43) Date of publication of application: **15.01.2020**
(21) Application number: 19185349.8
(22) Date of filing: 09.07.2019
(51) Int. Cl.: A61B 17/072

(54) **ADAPTER ASSEMBLIES FOR INTERCONNECTING ELECTROMECHANICAL HANDLE ASSEMBLIES AND SURGICAL LOADING UNITS**

(30) Priority: 10.07.2018 US 201862695876 P; 14.06.2019 US 201916441922
(71) Applicant: Covidien LP, Mansfield, MA 02048 (US)
(72) Inventor: PANTAZIS, John, Stratford, CT 06614 (US); CALDERONI, Anthony, Bristol, CT 06010 (US)
(74) Representative: Maschio & Soames IP Ltd

(57) **Abstract**

A surgical instrument includes a handle assembly, a giant magnetoresistance integrated circuit (GMR-IC), and an adapter assembly. The adapter assembly is configured to affect a function of a surgical loading unit in response to an actuation of the handle assembly. The adapter assembly includes a drive shaft and a magnetic material coupled to the drive shaft. In response to an actuation of the drive shaft by the handle assembly, the drive shaft is configured to move the magnetic material relative to the GMR-IC to change a magnetic field induced on the GMR-IC.

## Description

### CROSS-REFERENCE TO RELATED APPLICATIONS

This application claims the benefit of and priority to U.S. Provisional Patent Application No. 62/695,876 filed July 10, 2018, the entire disclosure of which is incorporated by reference herein.

### BACKGROUND

### 1. Technical Field

The present disclosure relates to adapter assemblies, for use with surgical instruments, that electrically and mechanically interconnect electromechanical handle assemblies and surgical loading units. More specifically, the present disclosure relates to adapter assemblies for surgical instruments that include force sensors for sensing an axial force output and/or input of the adapter assemblies.

### 2. Background of Related Art

A number of handle assembly manufacturers have developed product lines with proprietary drive systems for operating and/or manipulating electromechanical surgical instruments. In many instances, the electromechanical surgical instruments include a handle assembly, which is reusable, and disposable loading units and/or single use loading units or the like including an end effector that is selectively connected to the handle assembly prior to use and then disconnected from the handle assembly following use in order to be disposed of or in some instances sterilized for re-use.

In certain instances, it is desirable to measure a firing force produced by and/or transmitted through an adapter assembly positioned between the handle assembly and the loading unit. Measuring the firing force can be used, *inter alia,* to maintain the firing force within safe limits and to determine tissue thickness. Accordingly, a need exists for an adapter assembly capable of measuring its axial force output and/or input during operation thereof.

### SUMMARY

The present disclosure relates to adapter assemblies for use with surgical instruments that electrically and mechanically interconnect electromechanical handle assemblies and surgical loading units, and to force transmitting assemblies disposed within adapter assemblies that are configured to detect and measure an amount of axial force output and/or input of the adapter assembly.

In one aspect of the present disclosure, a surgical instrument is provided and includes a handle assembly, a giant magnetoresistance integrated circuit (GMR-IC), and an adapter assembly including a force transmitting assembly operably coupled to the handle assembly. The force transmitting assembly is configured to affect a function of a surgical loading unit in response to an actuation of the handle assembly. The force transmitting assembly includes a magnetic material coupled to a drive shaft. In response to an actuation of the drive shaft by the handle assembly, the drive shaft is configured to move the magnetic material relative to the GMR-IC to change a magnetic field induced on the GMR-IC.

In aspects, the adapter assembly may include a housing configured to be coupled to the handle housing of the handle assembly. The housing may have the magnetic material and the GMR-IC situated therein.

In aspects, the adapter assembly may include an outer tube having a proximal end portion supported by the housing.

In aspects, the GMR-IC may output a voltage that varies based on the magnetic field induced on the GMR-IC by the magnetic material.

In aspects, the surgical instrument may further include a processor configured to determine the force imparted on the drive shaft based on the voltage output by the GMR-IC.

In aspects, the handle assembly may further include a driving member operably coupled to a proximal end portion of the drive shaft of the adapter assembly.

In aspects, the adapter assembly may further include a distal drive member having a proximal end portion coupled to a threaded portion of the drive shaft, and a distal end portion configured to be operably coupled to a driven member of a surgical loading unit, such that rotation of the drive shaft longitudinally moves the distal drive member relative to the drive shaft to actuate the surgical loading unit.

In aspects, the GMR-IC may output a voltage that varies based on the magnetic field induced on the GMR-IC by the magnetic material. The handle assembly may further include a processor configured to determine a force experienced by the driven member of the surgical loading unit based on the voltage output by the GMR-IC.

In aspects, the adapter assembly may further include a printed circuit board and GMR-IC may be attached to the printed circuit board.

In aspects, the force transmitting assembly may further include a flange affixed to the drive shaft and the flange may include a magnetic material.

In aspects, the flange may extend laterally from the drive shaft.

In aspects, the flange may be spaced distally from the GMR-IC.

In another aspect of the present disclosure, an adapter assembly is provided for selectively interconnecting a surgical loading unit and a handle assembly that is configured to actuate the surgical loading unit. The adapter assembly includes a housing configured for selective connection with the handle assembly, a giant magnetoresistance integrated circuit (GMR-IC) disposed in the housing, an outer tube having a proximal end portion supported by the housing and a distal end portion configured to be coupled with the surgical loading unit, and a force transmitting assembly extending at least partially through the outer tube. The force transmitting assembly includes a drive shaft and a magnetic material coupled to the drive shaft. In response to an actuation of the drive shaft by the handle assembly, the drive shaft is configured to move the magnetic material relative to the GMR-IC to change a magnetic field induced on the GMR-IC.

### BRIEF DESCRIPTION OF THE DRAWINGS

Embodiments of the present disclosure are described herein with reference to the accompanying drawings, wherein:
FIG. 1A is a perspective view of a surgical instrument including an adapter assembly in accordance with an embodiment of the present disclosure interconnected between an exemplary electromechanical handle assembly and a surgical loading unit;
FIG. 1B is a perspective view illustrating an attachment of a proximal end portion of the adapter assembly to a distal end portion of the electromechanical handle assembly of FIG. 1A;
FIG. 2 is a cross-sectional view of the adapter assembly as taken along section line 2-2 of FIG. 1A;
FIG. 3 is perspective view of a force transmitting assembly of the adapter assembly of FIG. 2;
FIG 4 is an exploded view of the surgical loading unit of the surgical instrument 10 of FIG. 1A; and
FIG. 5 is a schematic diagram of a giant magnetoresistance integrated circuit sensing for linear force in accordance with the principles of the disclosure.

### DETAILED DESCRIPTION

Embodiments of the presently disclosed electromechanical surgical instruments including handle assemblies, adapter assemblies, and surgical loading units are described in detail with reference to the drawings, in which like reference numerals designate identical or corresponding elements in each of the several views. As used herein the term "distal" refers to that portion of the handle assembly, adapter assembly, surgical loading unit, or component thereof, farther from the user, while the term "proximal" refers to that portion of the handle assembly, adapter assembly, surgical loading unit, or component thereof, closer to the user.

A surgical instrument, in accordance with an embodiment of the present disclosure, is generally designated as 10, and is in the form of a powered hand held electromechanical surgical instrument configured for clamping and/or sealing tissue. The surgical instrument 10 includes a handle assembly 100, an adapter assembly 200, and a surgical loading unit 300. The handle assembly 100 is configured for selective coupling, via the adapter assembly 200, to a plurality of different surgical loading units, such as, for example, the surgical loading unit 300. Each surgical loading unit is configured for actuation and manipulation by the powered handle assembly 100.

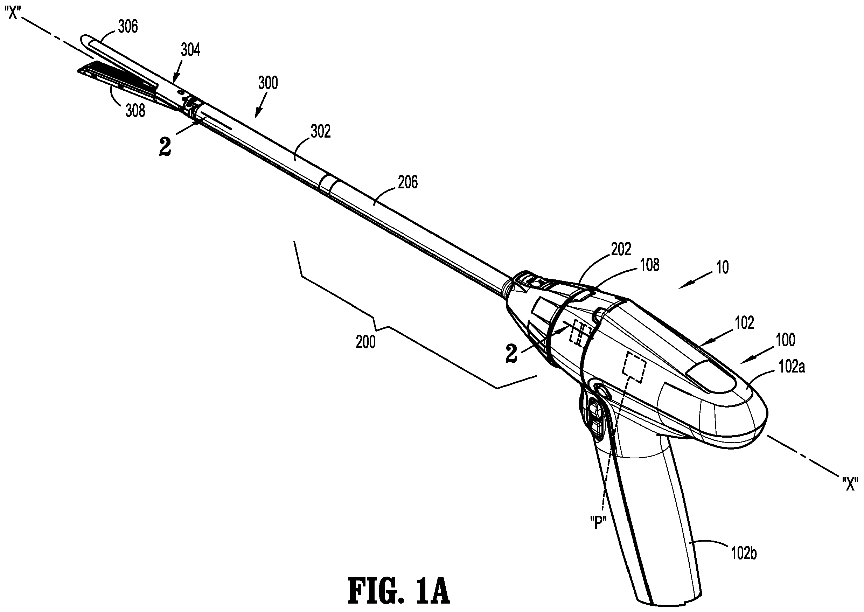

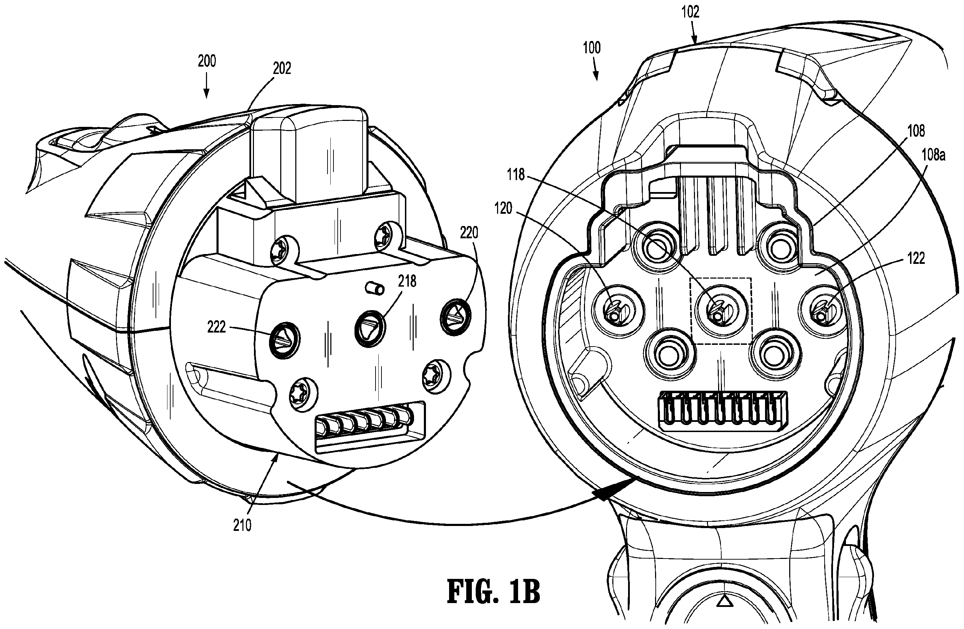

As illustrated in FIGS. 1A and 1B, the handle assembly 100 includes a handle housing 102 having an upper housing portion 102a which houses various components of the handle assembly 100, and a lower hand grip portion 102b extending from the upper housing portion 102a. The lower hand grip portion 102b may be disposed distally of a proximal-most end of the upper housing portion 102a. Alternately, other handle configurations are envisioned.

The handle housing 102 defines a cavity therein for selective removable receipt of a rechargeable battery (not shown) and receipt of a drive mechanism (not shown). The battery is configured to supply power to the electrical components of the surgical instrument 10. The cavity of the handle housing 102 has a processor "P" situated therein. The processor "P" is configured to control the various operations of the surgical instrument 10. The drive mechanism is configured to drive shafts and/or gear components in order to perform various operations of the surgical instrument 10. In particular, the drive mechanism is configured to drive shafts and/or gear components in order to selectively move a tool assembly 304 of the loading unit 300 relative to a proximal body portion 302 of the loading unit 300, to rotate the loading unit 300 about a longitudinal axis "X" relative to the handle assembly 100, to move/approximate an anvil assembly 306 and/or a cartridge assembly 308 of the loading unit 300 relative to one another, and/or to fire a stapling and cutting cartridge within the cartridge assembly 308 of the loading unit 300.

As illustrated in FIG. 1B, the handle housing 102 defines a connecting portion 108 configured to accept a corresponding drive coupling assembly 210 of the adapter assembly 200. Specifically, the connecting portion 108 of the handle assembly 100 has a recess 108a that receives a component of the drive coupling assembly 210 of the adapter assembly 200 when the adapter assembly 200 is mated to the handle assembly 100. The connecting portion 108 houses three rotatable, motorized drive connectors 118, 120, 122, which are arranged in a common plane or line with one another.

When the adapter assembly 200 is mated to the handle assembly 100, each of the rotatable drive connectors 118, 120, 122 of the handle assembly 100 couples with a corresponding rotatable connector sleeve 218, 220, 222 of the adapter assembly 200. In this regard, the interface between the corresponding first drive connector or driving member 118 and the first connector sleeve 218, the interface between the corresponding second drive connector 120 and the second connector sleeve 220, and the interface between the corresponding third drive connector 122 and the third connector sleeve 222 are keyed such that rotation of each of the drive connectors 118, 120, 122 of the handle assembly 100 causes a corresponding rotation of the corresponding connector sleeve 218, 220, 222 of the adapter assembly 200.

The mating of the drive connectors 118, 120, 122 of the handle assembly 100 with the connector sleeves 218, 220, 222 of the adapter assembly 200 allows rotational forces to be independently transmitted via each of the three respective connector interfaces. The drive connectors 118, 120, 122 of the handle assembly 100 are configured to be independently rotated by the drive mechanism of the handle assembly 100. In this regard, a function selection module (not shown) of the drive mechanism selects which drive connector or connectors 118, 120, 122 of the handle assembly 100 is to be driven by a motor (not shown) of the handle assembly 100.

Since each of the drive connectors 118, 120, 122 of the handle assembly 100 has a keyed and/or substantially non-rotatable interface with the respective connector sleeves 218, 220, 222 of the adapter assembly 200, when the adapter assembly 200 is coupled to the handle assembly 100, rotational force(s) are selectively transferred from the drive connectors of the handle assembly 100 to the adapter assembly 200. The selective rotation of the drive connector(s) 118, 120 and/or 122 of the handle assembly 100 allows the handle assembly 100 to selectively actuate different functions of the loading unit 300 (FIG. 1A). For example, selective and independent rotation of the first drive connector or rotatable driving member 118 of the handle assembly 100 may correspond to the selective and independent opening and closing of the tool assembly 304 (FIG. 1A) of the loading unit 300, and driving of a stapling/cutting component of the tool assembly 304 of the loading unit 300. As an additional example, the selective and independent rotation of the second drive connector 120 of the handle assembly 100 may correspond to the selective and independent articulation of the tool assembly 304 of the loading unit 300 transverse to a central longitudinal axis "X" (FIG. 1A). Additionally, for instance, the selective and independent rotation of the third drive connector 122 of the handle assembly 100 may correspond to the selective and independent rotation of the loading unit 300 about the longitudinal axis "X" (FIG. 1A) relative to the handle housing 102 of the handle assembly 100.

Reference may be made to International Application No. PCT/US2008/077249, filed September 22, 2008 (Inter. Pub. No. WO 2009/039506) and U.S. Patent Application No. 12/622,827, filed on November 20, 2009 (now U.S. Patent Publication No. 2011/0121049), the entire contents of each of which being incorporated herein by reference, for a detailed description of various internal components of and operation of exemplary electromechanical handle assembly 100.

With continued reference to FIGS. 1A and 1B, the adapter assembly 200 is configured for selectively interconnecting a surgical loading unit, for example, the surgical loading unit 300 and a handle assembly, for example, the handle assembly 100. The adapter assembly 200 is configured to convert a rotation of either of the drive connectors 118, 120 and 122 of the handle assembly 100 into axial translation useful for operating the loading unit 300.

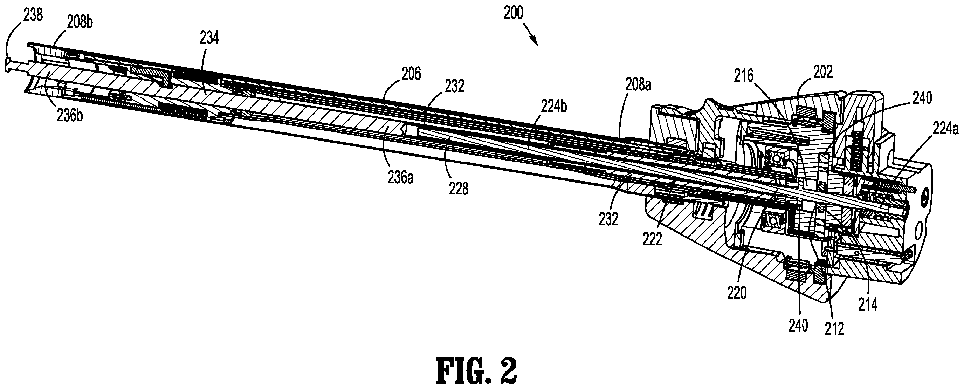

With reference to FIG. 2, the adapter assembly 200 generally includes a housing, such as, for example, a knob housing 202, and an outer tube 206 extending from a distal end portion of the knob housing 202. The knob housing 202 and the outer tube 206 are configured and dimensioned to house the components of the adapter assembly 200. The outer tube 206 is dimensioned for endoscopic insertion. In particular, the outer tube 206 is dimensioned to pass through a typical trocar port, cannula or the like. The knob housing 202 is dimensioned to prevent entry or passage of the knob housing 202 into the trocar port, cannula, or the like. The knob housing 202 is configured and adapted to connect to the connecting portion 108 (FIG. 1B) of the handle housing 102 of the handle assembly 100. The outer tube 206 has a proximal end portion 208a supported by the knob housing 202 and a distal end portion 208b configured to be selectively attached to the surgical loading unit 300 (FIG. 1A).

The housing 202 of the adapter assembly 200 includes a sensing element, such as, for example, a magnetoresistance integrated circuit ("GMR-IC") 212 attached to a printed circuit board 214. The GMR-IC 212 and the printed circuit board 214 may be disposed within a proximal end of the housing 202 and aligned with the drive connector 118 (FIG. 1B), and thus the central longitudinal axis "X" (FIG. 1A) defined by the outer tube 206. The GMR-IC 212 is configured to output a voltage commensurate with an axial force output by or input into the drive connector 118 during actuation thereof, and relay (e.g., wirelessly) the voltage to the processor "P" (FIG. 1A) of the handle assembly 100. The processor "P," in turn, is configured to calculate the axial force output by or input into the drive connector 118 based on the voltage output by the GMR-IC 212, as will be described in further detail herein.

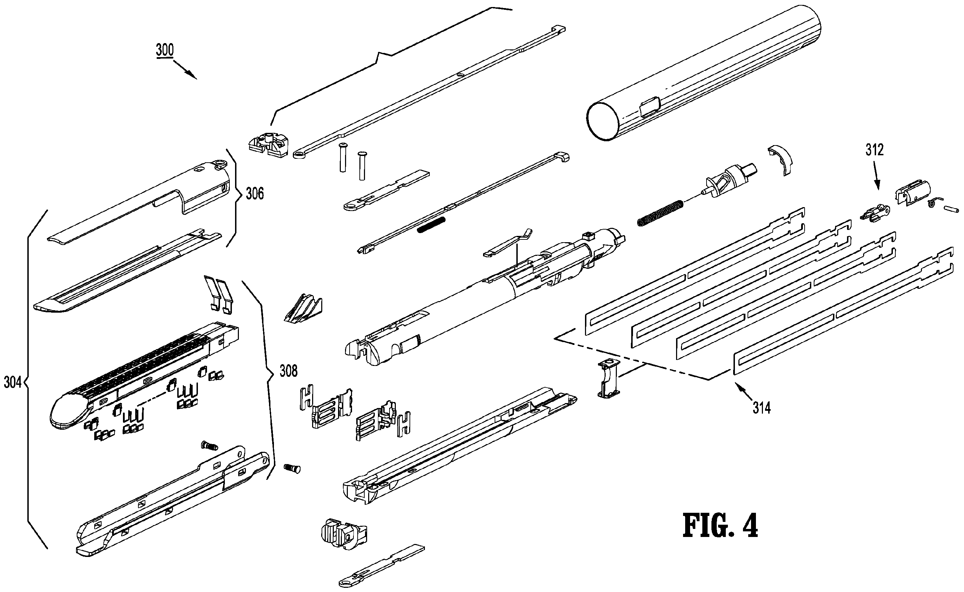

The adapter assembly 200 further includes a force/rotation transmitting/converting assembly 220 supported within the knob housing 202 and extending through the outer tube 206. The force transmitting assembly 220 is configured to transmit/convert a speed/force of rotation (e.g., increase or decrease) of the rotatable driving member 118 of the handle assembly 100 into an axial force before such rotational speed/force is transmitted to the surgical loading unit 300 (FIG. 1A). Specifically, the force transmitting assembly 220 is configured to transmit or convert a rotation of the driving member 118 (FIG. 1B) of the handle assembly 100 into axial translation of a translatable driven member 312 (FIG. 4) of the surgical loading unit 300 to effectuate articulation, closing, opening and/or firing of the loading unit 300.

Reference may be made to U.S. Patent No. 7,819,896, filed on August 31, 2009, entitled "TOOL ASSEMBLY FOR A SURGICAL STAPLING DEVICE" for a detailed discussion of the construction and operation of the loading unit 300, as illustrated in FIG. 4.

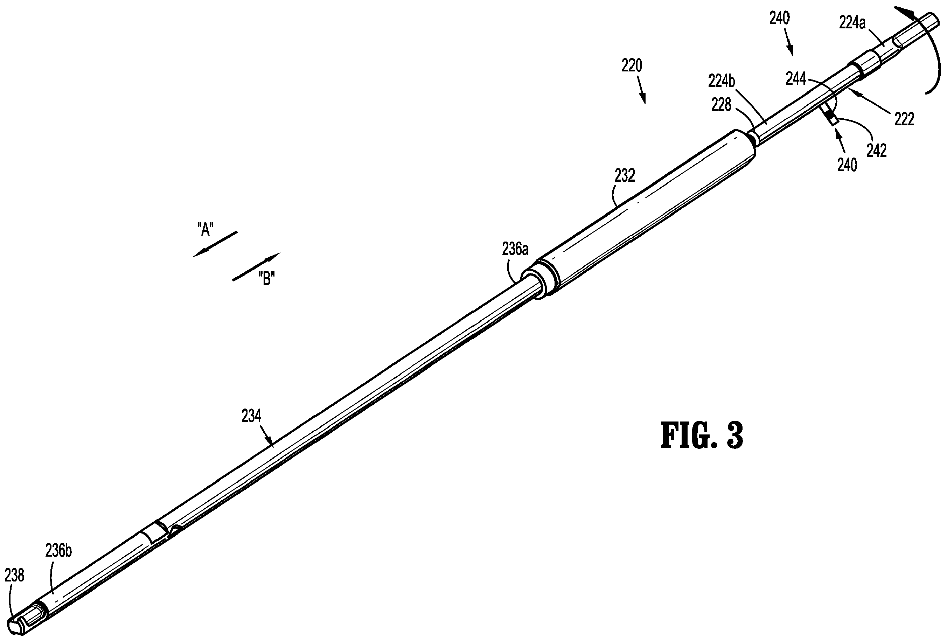

With reference to FIGS. 2 and 3, the force transmitting assembly 220 includes a rotatable drive shaft 222, a drive coupling nut 232 rotatably coupled to a threaded portion 228 of the drive shaft 222, and a distal drive member 234. The drive shaft 222 includes a proximal portion 224a configured to be operatively coupled to the driving member 118 (FIG. 1B) of the handle assembly 100 via the first connector sleeve 218 (FIG. 1B). The drive coupling nut 232 is slidably disposed within and slidably keyed to the outer tube 206 so as to be prevented from rotating as the drive shaft 222 is rotated. In this manner, as the drive shaft 222 is rotated, the drive coupling nut 232 is translated along the threaded portion 228 of the drive shaft 222 and, in turn, through and/or along the outer tube 206.

The distal drive member 234 of the force transmitting assembly 220 has a proximal end portion 236a coupled to a distal end portion 224b of the drive shaft 222 via mechanical engagement with the drive coupling nut 232, such that axial movement of the drive coupling nut 232 results in a corresponding amount of axial movement of the distal drive member 234. The distal drive member 234 has a distal end portion 236b configured to be operatively coupled to the translatable driven member 312 (FIG. 4) of the surgical loading unit 300. In particular, the distal end portion 236b of the distal drive member 234 supports a connection member 238 configured and dimensioned for selective engagement with the translatable driven member 312 (FIG. 4) of the loading unit 300. The drive coupling nut 232 and/or the distal drive member 234 function as a force transmitting member to components of the loading unit 300.

In use, as the drive shaft 222 is rotated, due to a rotation of the first connector sleeve 218, as a result of the rotation of the rotatable driving member 118 of the handle assembly 100, the drive coupling nut 232 is caused to be translated within the outer tube 206. As the drive coupling nut 232 is caused to be translated axially, the distal drive member 234 is caused to be translated axially within the outer tube 206. As the distal drive member 234 is translated axially in a distal direction, with the connection member 238 connected thereto and engaged with the translatable driven member 312 of a drive assembly 314 of the loading unit 300 (FIG. 4), the distal drive member 234 causes concomitant axial translation of the translatable driven member 312 of the loading unit 300 to effectuate a closure and a firing of the tool assembly 304 of the loading unit 300.

With specific reference to FIGS. 2 and 3, the force transmitting assembly further includes a sensed element 240 designed and adapted to move in response to axial movement of the drive shaft 222, wherein said movement is sensed by the GMR-IC 212 to determine an axial force output from and/or input into the drive shaft 222 of the adapter assembly 200, as will be described. The sensed element 240 is coupled to the drive shaft 222 such that proximal, and in embodiments, distal, longitudinal movement of the drive shaft 222 moves the sensed element 240 in a corresponding direction.

The sensed element 240 includes a flange 242, such as, for example, a metal flange, and a magnetic material 244, such as, for example, a permanent magnet, coupled to the flange 242. The flange 242 extends laterally from the drive shaft 222 (e.g., perpendicularly) and is spaced distally from the GMR-IC 212, such that the flange 242 and the GMR-IC have a cavity 216 defined therebetween. In embodiments, the flange 242 of the sensed element 240 may be spaced proximally from the GMR-IC 212. The flange 242 is fixed to the drive shaft 222, such that proximal, longitudinal movement of the drive shaft 222 along the longitudinal axis "X" moves the flange 242 therewith. In an alternative embodiment, instead of the flange 242 extending from one side of the drive shaft 222, the flange 242 may define a channel extending therethrough having the drive shaft 222 received therein.

The magnet 244 of the sensed element 240 is embedded in the flange 242. In embodiments, the magnet 244 may be disposed or coated on an outer surface of the flange 242 rather than be embedded within the flange 242. In other embodiments, the flange 242 may be fabricated from a magnetic material instead of having a magnet coupled thereto. As the sensed element 240 moves with the drive shaft 222 during actuation of the end effector 300 (FIG. 1A), the magnet 244 moves relative to the GMR-IC 212, whereby the magnetic field induced on the GMR-IC 212 by the magnet 244 is changed. The GMR-IC 212 outputs a voltage that varies based on the magnetic field induced thereon by the magnet 244, whereby the GMR-IC 212 relays (e.g., wirelessly) the voltage to the processor "P" (FIG. 1A) of the handle assembly 100. The processor "P" then correlates the voltage output by the GMR-IC 212 with an amount of axial force output by the drive shaft 222 of the adapter assembly 200.

In operation, the GMR-IC 212 and the magnet 244 of the sensed element 240 cooperate to detect and measure an axial force output by the adapter assembly 200 during operation of the handle assembly 100. The handle assembly 100 is actuated to carry out various functions of the surgical loading unit 300. As the handle assembly 100 is actuated, the drive shaft 222 of the force transmitting assembly 220 is rotated relative to the coupling nut 232 to axially move the coupling nut 232 in a distal direction relative to the drive shaft 222. Distal movement of the coupling nut 232 longitudinally moves the distal drive member 234 of the force transmitting assembly 220 relative to the drive shaft 222 resulting in a force, applied in a direction indicated by arrow "A" in FIG. 3, to the translatable driven member 312 (FIG. 4) of the surgical loading unit 300. An equal and opposite reactive force is exerted by the translatable driven member 312 of the surgical loading unit 300, in a direction indicated by arrow "B" in FIG. 3, on the distal drive member 234. The reactive force exerted on the distal drive member 234 is transmitted in a proximal direction along the force transmitting assembly 220 to the sensing element 240 (the flange 242 and the attached magnet 244).

Due to the GMR-IC 212 being disposed proximally adjacent the magnet 244, the magnetic field induced on the GMR-IC 212 by the moving magnet 244 changes in a predictable manner based on the distance traveled by the magnet 244. The GMR-IC 212 outputs a voltage corresponding to the change in the magnetic field of the magnet 244. The voltage output by the GMR-IC 212 is communicated to the processor "P," (FIG. 1A) whereby the processor "P," using the voltage calculates the axial force output by the adapter assembly 200.

Knowing the amount of axial force output by the adapter assembly 200 can be used, *inter alia,* to prevent further actuation of the loading unit 300 upon reaching a threshold amount of axial output force deemed unsafe, to determine the amount of force needed to retract the drive assembly 314 of the loading unit 300 (FIG. 4) after actuating the loading unit 300, and/or to measure the amount of force needed to clamp tissue so as to determine tissue thickness, which can allow a clinician to determine whether tissue is too thick or too thin for a particular surgical loading unit. The information made available by the GMR-IC 212 and sensed element 240 can also be used to indicate to a clinician that the drive assembly 314 of the loading unit 300 (FIG. 4) has reached an end or a stop of the loading unit 300 or a firing sled of the loading unit 300 has reached an end or stop of a staple cartridge 308. Reference may be made to U.S. Patent No. 8,517,241, filed March 3, 2011, the entire contents of which is incorporated herein by reference, for a more detailed description of uses of said information provided by the GMR-IC 212 and the sensed element 240.

In embodiments, the GMR-IC may be replaced with a hall effect sensor.

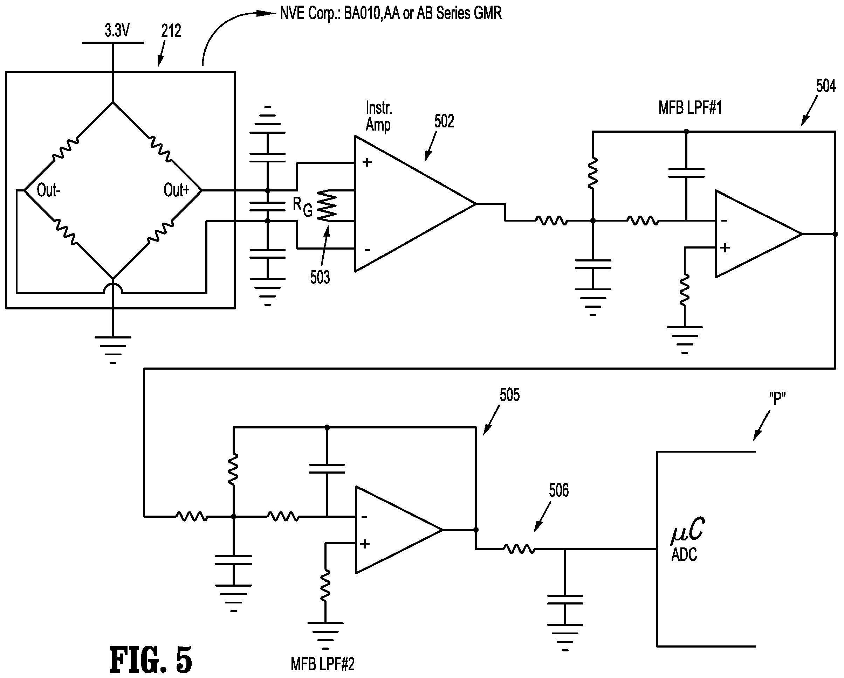

The circuit shown in FIG. 5 represents a method to adapt the GMR-IC 212 to an A/D input of a microprocessor or processor "P." The GMR-IC 212 may act like a Wheatstone bridge activated by strength of a local magnetic field. The GMR-IC 212 is powered by DC power and a differential signal resulting from a local magnetic field is amplified by an instrumentation amplifier 502 having a gain setting resistor 503. The resulting instrumentation amplifier 502 output is further amplified and filtered (low-pass) by second order active filters 504 and 505. A single pole low pass filter 506 further filters the amplified signal for presentation to the A/D input of the microprocessor "P." Further signal conditioning and filtering may take place internal to the microprocessor "P." The microprocessor "P" can have both offset and gain correction factors. Automatic offset correction can be achieved by recognition of an open jaw condition of the surgical instrument 10 (FIG. 1) where one would expect that no force is applied to the jaws 306, 308. At an appropriate time under this condition of zero force application, the microprocessor "P" may take an offset measurement and use that zero condition measurement to subtract from the signal presented to the A/D converter as an offset correction factor. This can be done each time the surgical instrument 10 is used, thereby automatically compensating for offset drift that may occur. The gain calibration for individual GMR-IC sensors may be communicated to the microprocessor "P" through 1-WIRE EEPROMs as part of the GMR-IC 212 or other suitable means.

Any of the components described herein may be fabricated from either metals, plastics, resins, composites or the like taking into consideration strength, durability, wearability, weight, resistance to corrosion, ease of manufacturing, cost of manufacturing, and the like.

It will be understood that various modifications may be made to the embodiments of the presently disclosed adapter assemblies. It is envisioned that the elements and features illustrated or described in connection with one exemplary embodiment may be combined with the elements and features of another without departing from the scope of the present disclosure. Therefore, the above description should not be construed as limiting, but merely as exemplifications of embodiments. Those skilled in the art will envision other modifications within the scope and spirit of the present disclosure.

The invention may be described by reference to the following numbered paragraphs:-
1. A surgical instrument, comprising:
   a handle assembly;
   a giant magnetoresistance integrated circuit (GMR-IC); and
   an adapter assembly including a force transmitting assembly operably coupled to the handle assembly and configured to affect a function of a surgical loading unit in response to an actuation of the handle assembly, the force transmitting assembly including:
      a drive shaft; and
      a magnetic material coupled to the drive shaft, wherein the drive shaft is configured to move the magnetic material in response to an actuation thereof by the handle assembly, such that the magnetic material moves relative to the GMR-IC to change a magnetic field induced on the GMR-IC.
2. The surgical instrument according to paragraph 1, wherein the adapter assembly includes a housing configured to be coupled to the handle housing of the handle assembly, the housing having the magnetic material and the GMR-IC situated therein.
3. The surgical instrument according to paragraph 2, wherein the adapter assembly includes an outer tube having a proximal end portion supported by the housing.
4. The surgical instrument according to paragraph 1, wherein the GMR-IC outputs a voltage that varies based on the magnetic field induced on the GMR-IC by the magnetic material.
5. The surgical instrument according to paragraph 4, further comprising a processor configured to determine the force imparted on the drive shaft based on the voltage output by the GMR-IC.
6. The surgical instrument according to paragraph 1, wherein the handle assembly further includes a driving member operably coupled to a proximal end portion of the drive shaft of the adapter assembly.
7. The surgical instrument according to paragraph 1, wherein the adapter assembly further includes a distal drive member having:
   a proximal end portion coupled to a threaded portion of the drive shaft; and
   a distal end portion configured to be operably coupled to a driven member of a surgical loading unit, such that rotation of the drive shaft longitudinally moves the distal drive member relative to the drive shaft to actuate the surgical loading unit.
8. The surgical instrument according to paragraph 7, wherein the GMR-IC outputs a voltage that varies based on the magnetic field induced on the GMR-IC by the magnetic material, the handle assembly further including a processor configured to determine a force experienced by the driven member of the surgical loading unit based on the voltage output by the GMR-IC.
9. The surgical instrument according to paragraph 1, wherein the adapter assembly further includes a printed circuit board, the GMR-IC being attached to the printed circuit board.
10. The surgical instrument according to paragraph 1, wherein the force transmitting assembly further includes a flange affixed to the drive shaft, the flange having the magnetic material disposed therewith.
11. The surgical instrument according to paragraph 10, wherein the flange extends laterally from the drive shaft.
12. The surgical instrument according to paragraph 10, wherein the flange is spaced distally from the GMR-IC.
13. An adapter assembly for selectively interconnecting a surgical loading unit and a handle assembly that is configured to actuate the surgical loading unit, the adapter assembly comprising:
   a housing configured for selective connection with a handle assembly;
   a giant magnetoresistance integrated circuit (GMR-IC) disposed in the housing;
   an outer tube having a proximal end portion supported by the housing and a distal end portion configured to be coupled with a surgical loading unit; and
   a force transmitting assembly extending at least partially through the outer tube and including:
      a drive shaft; and
      a magnetic material coupled to the drive shaft, wherein the drive shaft is configured to move the magnetic material in response to an actuation thereof by the handle assembly, such that the magnetic material moves relative to the GMR-IC to change a magnetic field induced on the GMR-IC.
14. The adapter assembly according to paragraph 13, further comprising a distal drive member including:
   a proximal end portion coupled to a threaded portion of the drive shaft; and
   a distal end portion configured to be operably coupled to a driven member of a surgical loading unit, such that rotation of the drive shaft longitudinally moves the distal drive member relative to the drive shaft to actuate the surgical loading unit.
15. The adapter assembly according to paragraph 13, wherein the GMR-IC outputs a voltage that varies based on the magnetic field induced on the GMR-IC by the magnetic material.
16. The adapter assembly according to paragraph 15, further comprising a printed circuit board, the GMR-IC being attached to the printed circuit board.
17. The adapter assembly according to paragraph 13, wherein the force transmitting assembly further includes a flange affixed to the drive shaft, the flange having the magnetic material disposed therewith.
18. The adapter assembly according to paragraph 17, wherein the flange extends laterally from the drive shaft.
19. The adapter assembly according to paragraph 17, wherein the flange is spaced distally from the GMR-IC.

## Claims

1. A surgical instrument, comprising:
a handle assembly;
a giant magnetoresistance integrated circuit (GMR-IC); and
an adapter assembly including a force transmitting assembly operably coupled to the handle assembly and configured to affect a function of a surgical loading unit in response to an actuation of the handle assembly, the force transmitting assembly including:
a drive shaft; and
a magnetic material coupled to the drive shaft, wherein the drive shaft is configured to move the magnetic material in response to an actuation thereof by the handle assembly, such that the magnetic material moves relative to the GMR-IC to change a magnetic field induced on the GMR-IC.

2. The surgical instrument according to claim 1, wherein the adapter assembly includes a housing configured to be coupled to the handle housing of the handle assembly, the housing having the magnetic material and the GMR-IC situated therein.

3. The surgical instrument according to claim 2, wherein the adapter assembly includes an outer tube having a proximal end portion supported by the housing.

4. The surgical instrument according to any preceding claim, wherein the GMR-IC outputs a voltage that varies based on the magnetic field induced on the GMR-IC by the magnetic material; preferably further comprising a processor configured to determine the force imparted on the drive shaft based on the voltage output by the GMR-IC.

5. The surgical instrument according to any preceding claim, wherein the handle assembly further includes a driving member operably coupled to a proximal end portion of the drive shaft of the adapter assembly.

6. The surgical instrument according to any preceding claim wherein the adapter assembly further includes a distal drive member having:
a proximal end portion coupled to a threaded portion of the drive shaft; and
a distal end portion configured to be operably coupled to a driven member of a surgical loading unit, such that rotation of the drive shaft longitudinally moves the distal drive member relative to the drive shaft to actuate the surgical loading unit; preferably wherein the GMR-IC outputs a voltage that varies based on the magnetic field induced on the GMR-IC by the magnetic material, the handle assembly further including a processor configured to determine a force experienced by the driven member of the surgical loading unit based on the voltage output by the GMR-IC.

7. The surgical instrument according to any preceding claim, wherein the adapter assembly further includes a printed circuit board, the GMR-IC being attached to the printed circuit board.

8. The surgical instrument according to any preceding claim, wherein the force transmitting assembly further includes a flange affixed to the drive shaft, the flange having the magnetic material disposed therewith.

9. The surgical instrument according to claim 8, wherein the flange extends laterally from the drive shaft.

10. The surgical instrument according to claim 8, wherein the flange is spaced distally from the GMR-IC.

11. An adapter assembly for selectively interconnecting a surgical loading unit and a handle assembly that is configured to actuate the surgical loading unit, the adapter assembly comprising:
a housing configured for selective connection with a handle assembly;
a giant magnetoresistance integrated circuit (GMR-IC) disposed in the housing;
an outer tube having a proximal end portion supported by the housing and a distal end portion configured to be coupled with a surgical loading unit; and
a force transmitting assembly extending at least partially through the outer tube and including:
a drive shaft; and
a magnetic material coupled to the drive shaft, wherein the drive shaft is configured to move the magnetic material in response to an actuation thereof by the handle assembly, such that the magnetic material moves relative to the GMR-IC to change a magnetic field induced on the GMR-IC.

12. The adapter assembly according to claim 11, further comprising a distal drive member including:
a proximal end portion coupled to a threaded portion of the drive shaft; and
a distal end portion configured to be operably coupled to a driven member of a surgical loading unit, such that rotation of the drive shaft longitudinally moves the distal drive member relative to the drive shaft to actuate the surgical loading unit.

13. The adapter assembly according to claim 11 or 12 wherein the GMR-IC outputs a voltage that varies based on the magnetic field induced on the GMR-IC by the magnetic material.

14. The adapter assembly according to any of claims 11 to 13, further comprising a printed circuit board, the GMR-IC being attached to the printed circuit board; preferably wherein the force transmitting assembly further includes a flange affixed to the drive shaft, the flange having the magnetic material disposed therewith.

15. The adapter assembly according to any of claims 11 to 14, wherein the flange extends laterally from the drive shaft; and/or wherein the flange is spaced distally from the GMR-IC.
